**Europäisches Patentamt**

(19) **European Patent Office**

**Office européen des brevets**

(11) Veröffentlichungsnummer: **0 036 406**
**B1**

(12) # EUROPÄISCHE PATENTSCHRIFT

(45) Veröffentlichungstag der Patentschrift:
**21.08.85**

(51) Int. Cl.⁴: **C 07 C 51/48, C 07 C 51/46,**
**C 07 C 53/02, C 07 C 53/08,**
**C 07 D 307/50**

(21) Anmeldenummer: **81890031.8**

(22) Anmeldetag: **24.02.81**

(54) Verfahren zur Extraktion von Essigsäure, Ameisensäure, gegebenenfalls Furfurol.

(30) Priorität: **14.03.80 AT 1423/80**

(43) Veröffentlichungstag der Anmeldung:
**23.09.81 Patentblatt 81/38**

(45) Bekanntmachung des Hinweises auf die Patenterteilung:
**21.08.85 Patentblatt 85/34**

(84) Benannte Vertragsstaaten:
**BE CH DE FR GB IT LI NL SE**

(56) Entgegenhaltungen:
**DE - A - 2 423 272**
**DE - A - 2 700 212**
**DE - A - 2 926 520**
**US - A - 3 801 629**

**CHEMIE-INGENIEUR-TECHNIK, Band 38, Nr. 10, Oktober 1966, Weinheim, W. HUNSMANN et al. "Trennung von Wasser, Ameisensäure und Essigsäure durch Azeotrop-Destillation", Seiten 1053 bis 1059**

(73) Patentinhaber: **VEREINIGTE EDELSTAHLWERKE AKTIENGESELLSCHAFT (VEW), Elisabethstrasse 12, A-1010 Wien (AT)**

(72) Erfinder: **Kanzler, Walter, Dipl.-Ing.,**
**Viktor-Geramb-Weg 13, A-8010 Graz (AT)**
Erfinder: **Schedler, Johannes, Dipl.-Ing.,**
**Rupertistrasse 119, A-8042 Graz - St. Peter (AT)**

(74) Vertreter: **Widtmann, Georg, Dr., Elisabethstrasse 12, A-1010 Wien (AT)**

## Beschreibung

Die Erfindung bezieht sich auf ein Verfahren zur Extraktion von Essigsäure, Ameisensäure, gegebenenfalls Furfurol u. dgl. aus einer wäßrigen Lösung von pflanzlichen Hydrolyseprodukten.

Beim Aufschluß von Pflanzenmaterial und deren Abfällen entstehen neben Mono-, Oligo- und Polysacchariden größere Mengen an niedermolekularen organischen Verbindungen verschiedener Art, wie niedere organische Säuren, Ketone, Aldehyde und Alkohole. Der Aufschluß erfolgt durch Einwirkung von Säuren, Basen, Alkoholen, Sauerstoff u. a. bei Temperaturen bis über 250°C in wäßrigem Milieu. Je nach den herrschenden Bedingungen ist dieser Aufschluß vollständig oder es wird nur der Zellstoff aus den Lignozellulosen freigesetzt. Dabei entsteht immer eine verdünnte, wäßrige Lösung, in der die aus den Pflanzen und Pflanzenabfällen herausgelösten und abgebauten Substanzen enthalten sind. Dabei wird meist die Gewinnung nur eines Teiles der nach dem Aufschluß vorliegenden Substanzen angestrebt. Die Lösung mit den nicht genutzten Substanzen wird meist eingedickt und verbrannt und die Brüdenkondensate dem Vorfluter zugeleitet. Es hat sich aber gezeigt, daß die flüchtigen Substanzen zum Großteil in die Brüdenkondensate übergehen und somit die Vorfluter belasten. Solche Hydrolysate entstehen bei der Zellstoffgewinnung, der Furfurolgewinnung, der Holzverzuckerung u. a. Bisher wurden solche Hydrolysate oder Brüdenkondensate durch Behandlung mit leicht flüchtigen, polaren Lösungsmitteln oder mit Wasserdampf behandelt.

Diese Verfahren sind sehr energieaufwendig und daher in den meisten Anwendungsfällen nicht wirtschaftlich anzuwenden.

Aus der DE-OS 2 423 272 wird ein Verfahren zur Gewinnung von Karbonsäuren aus wäßrigen Lösungen bekannt, wobei diese durch Extraktion mit einem mindest 1 gew.-%igen polaren Extraktionsmittel, wie Dialkylalkylphosphate, Alkyldialkylphosphinate, Trialkylphosphinoxide mit 10 bis 36 Kohlenstoffatomen pro Molekül, Dialkyl-alicyclische-amidophosphate, Dialkylsulfoxide bekannt, wobei als Verdünnungsmittel halogenierte Kohlenwasserstoffe, Silane, Kerosin u. dgl. vorgeschlagen werden. Die Extraktion erfolgt bei Raumtemperatur. Die Aufarbeitung des beladenen Extraktionsmittels erfolgt durch Abstreifen mit einer wäßrigen Baselösung oder mit gasförmigem Ammoniak. Sollen die Säuren gewonnen werden, so ist dann eine aufwendige Aufarbeitung des Saltgemisches erforderlich, wodurch eine wirtschaftliche Gewinnung der Säuren schwer möglich ist.

In der DE-OS 2 926 520 wird ein Verfahren zur Abtrennung von umweltschädlichen Geruchsstoffen bzw. Essigsäure, Ameisensäure und Furfurol aus einem Brüdenkondensator beschrieben, wobei mit einer 30%igen Trioctylphosphinoxidlösung bei 80°C die angeführten Stoffe extrahiert werden, wonach die extrahierten Stoffe durch Destillation vom Extraktionsmittel getrennt werden. Durch diese Vorgangsweise ist zwar eine gute Abtrennung aus der wäßrigen Lösung möglich und es kann durch die destillative Abtrennung der Schadstoffe das Extraktionsmittel schnell wieder eingesetzt werden, jedoch ist dort eine Aufarbeitung des abgetrennten Destilates nicht näher beschrieben und wirtschaftlich nur schwer möglich.

Aus der DD-PS 112 078 wird ein Verfahren zur destillativen Abtrennung von Wasser aus Essigsäure und Ähtylacetat bzw. Vinylacetat durch Azeotropdestillation dieser Komponenten mit dem enthaltenen Wasser bekannt.

Aus Chemie-Ingenieur-Technik, 38 Nr. 10, S. 1053—1059 (1966) ist bekannt, daß azeotrope Gemische der eingangs erwähnten Stoffe weniger als 50 Mol.% Wasser enthalten, woraus gefolgert werden kann, daß bei kontinuierlicher Rektifikation nacheinander Wasser und ein Gemisch bzw. Azeotrop abdestillieren. Diese Literaturstelle betrifft allerdings ausschließlich ein ternäres System mit den reinen Komponenten Ameisensäure, Essigsäure und Wasser und kann zur Abschätzung des tatsächlichen Verhaltens der oben angeführten, aus Pflanzenhydrolysaten stammenden, wesentlich komplexeren Gemische nichts beitragen.

Das erfindungsgemäße Verfahren zur Extraktion von Essigsäure, Ameisensäure, gegebenenfalls Furfurol u. dgl. aus einer wäßrigen Lösung von pflanzlichen Hydrolyseprodukten, wobei mit flüssigem, im wesentlichen wasserunlöslichem und somit mit Wasser unmischbarem Trialkylphosphinoxid, das gegebenenfalls in einem organischen, im wesentlichen wasserunlöslichen Lösungsmittel gelöst ist, die Essigsäure, Ameisensäure und gegebenenfalls das Furfurol und dgl. bei einer Temperatur zwischen 40° und 100°C aus der wäßrigen Lösung extrahiert und von dem Extraktionsmittel durch Destillation getrennt werden, besteht im wesentlichen darin, daß ein Lösungsmittel für das Extraktionsmittel, das im Bereich von 170 bis 220°C bei Normaldruck siedet, verwendet wird, daß in einer ersten Stufe der überwiegende Teil des im beladenen Extraktionsmittel gelösten Wassers abdestilliert wird, und daß, vorzugsweise bei Unterdruck, in einer zweiten Stufe das Lösungsmittel für das Extraktionsmittel zusammen mit einer Mischung, vorzugsweise einem Azeotrop, mit Essigsäure, Ameisensäure, Wasser, gegebenenfalls Furfurol und Verunreinigungen abdestilliert wird, und unbeladenes Extraktionsmittel abgezogen wird. Durch die erfindungsgemäße Kombination von Maßnahmen und Bedingung ist erstmals eine wirtschaftliche Gewinnung der Säuren sowie des Furfurols möglich, da in einer ersten Stufe ein Großteil des Wassers abgetrennt wird, so daß dieses bei der Aufarbeitung des Gemisches nicht mehr so störend wirkt, wobei ein trockenes und sofort wieder einsetzbares Extraktionsmittel durch Destillation der Säuren und des Furfurols sowie des Lösungsmittels zurückgewonnen wird, und eine Zersetzung des Extraktionsmittels vermieden werden kann. Die als Destillat gewonnene Mischung mit Essigsäure, Ameisensäure und Furfurol kann auf Grund des geringen Wassergehaltes besonders leicht aufgearbeitet werden. Da schon in der zweiten Stufe Extraktionsmittel abgezogen wird, wird es geschont und belastet die weitere Aufarbeitung nicht mehr.

Gemäß einem weiteren Merkmal der Erfindung wird als Extraktionsmittel Trioctylphosphinoxid und als Lösungsmittel ein aliphatischer Kohlenwasserstoff, vorzugsweise Undecan, verwendet. Diese Kombination ist besonders auf Grund der selektiven Extraktionseigenschaften und der geringen Löslichkeit in Wasser, sowie ihrer destillativen Eigenschaften, wie Siedebereich, Mischungslücken usw. besonders geeignet.

Gemäß einem weiteren Merkmal der Erfindung wird eine 20 bis 80 gew.-%ige, vorzugsweise 30 bis 40 gew.-%ige, Lösung von Trioctylphosphinoxid, vorzugsweise in n-Undecan, verwendet. Wird eine niedrigprozentigere Lösung verwendet, so ist auch bei einer gegenüber der Raumtemperatur erhöhten Extraktionstemperatur der Stoffübergang niedrig, so daß große Mengen Extraktionsmittel verwendet werden müssen, wohingegen bei einer zu hochprozentigen Lösung der Effekt auf Grund der steigenden Viskosität der Lösung, sowie des zu hohen erforderlichen Vakuums, bei der Abtrennung der Produktmischung mit den Lösungsmitteln von Nachteil ist.

Für die weitere Aufarbeitung der von dem Extraktionsmittel abgetrennten Mischung mit Essigsäure, Ameisensäure, Wasser und gegebenenfalls Furfurol hat es sich als vorteilhaft erwiesen, wenn vorhandenes Methanol und weitere Verunreinigungen abdestilliert werden.

Wird dem Gemisch mit Essigsäure, Ameisensäure, Furfurol und Wasser ein Schleppmittel aus Di-n-propyläther und/oder Äthyl-n-butyläther für das Wasser zugegeben, worauf das Wasser mit dem Schleppmittel abdestilliert wird, so kann auf besonders einfache und wirtschaftliche Art und Weise eine wasserfreie Mischung erhalten werden, die sodann einer einfachen destillativen Trennung zugeführt werden kann, wobei überraschenderweise das Furfurol nicht gemeinsam mit dem Wasser abgetrennt wird.

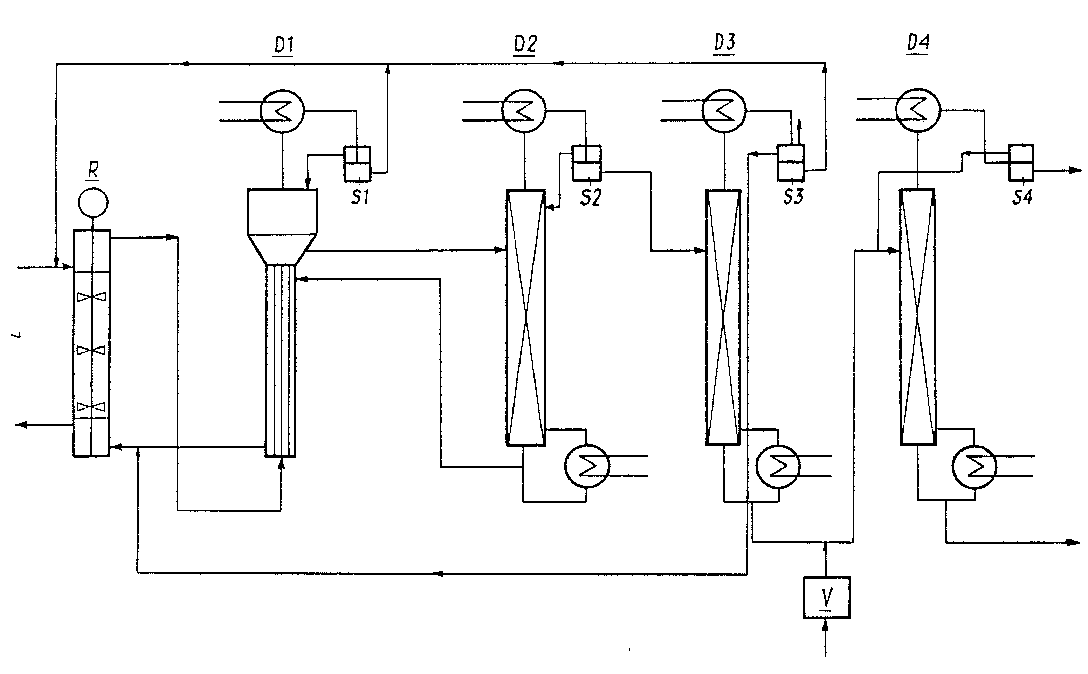

Im folgenden wird die Erfindung an Hand des in der Zeichnung dargestellten Fließschemas näher erläutert:

Ein Brüdenkondensat aus der Zellstoffproduktion aus Laubbäumen mit 2 Vol.-% Inhaltsstoffen (Essigsäure 1,5%, Ameisensäure 0,1%, Furfurol 0,4%) wird mit derselben Menge einer Lösung von 30 Gew.-% Trioctylphosphinoxid in n-Undekan bei 80°C extrahiert. Es kann für das erfindungsgemäße Verfahren auch ein anderes Lösungsmittel verwendet werden, das im Bereich von 170° bis 220°C bei Normaldruck siedet, wobei aliphatische Kohlenwasserstoffe besonders geeignet sind. 100 m³/h Brüdenkondensat werden in den Rührextraktor R oben eingeleitet. Dabei ist wichtig, daß das Brüdenkondensat klar und frei von Laugeresten ist. Weiters werden 100 m³/h des Extraktionsmittelgemisches in den Extraktor R jedoch unten eingeleitet. Die Phasenführung in dem Extraktor wird von Hand eingestellt.

Die Koaleszenzfläche soll in den oberen Absetzkammern liegen, wobei das Brüdenkondensat die kontinuierliche Phase bildet.

Die Klarheit des gereinigten Brüdenkondensates wird mit einer Fotozelle (nicht dargestellt) überwacht. Im Falle einer Trübung durch Emulsionsbildung ertönt ein Alarmzeichen, Brüdenkondensatzulauf, Extraktionsmittelzulauf und Drehzahl des Rohrextraktors werden gedrosselt und die Emulsion durch einen Separator (nicht dargestellt) gespalten.

Aus dem Rührextraktor wird ein Brüdenkondensat abgeleitet, das weitgehend frei von Essigsäure, Ameisensäure, Methanol, $SO_2$ und Furfurol ist.

Das mit den Inhaltsstoffen des Brüdenkondensates beladene Extraktionsmittel wird im Rekuperator D1 mit dem aus der Kolonne D2 zurückfließenden Extraktionsmittel auf ca. 120°C aufgewärmt, wobei ca 65 Gew.-% des im Extraktionsmittel gelösten Wassers ausgetrieben werden und die wäßrigen Brüden, die geringe Mengen an Inhaltsstoffen enthalten, werden über den Scheider S1 wieder dem Extraktor R zugeführt.

Die im Scheider S1 abgetrennte organische Phase geht in die Kolonne D1 zurück. Das beladene Extraktionsmittel gelangt in die Kolonne D2, wo im Vakuumbetrieb bei ca. 0,2 bar bei 140°C ein Azeotrop aus Essigsäure, Ameisensäure, Furfurol, Wasser, Undekan und Verunreinigungen ($SO_2$, Methanol u. a.) abdestilliert wird.

Die kondensierten Kopfprodukte zerfallen im Scheider S2 in eine wäßrige und eine organische Phase. Dabei wird die organische Phase als Rücklauf in die Kolonne D2 rückgeführt und die wäßrige Phase enthält die Produkte. Das im Sumpf verbleibende reine Extraktionsmittel dient als Wärmeträger in der Kolonne D1 und wird dann dem Extraktor zugeführt.

Die Wirkung der Strippung in der Kolonne D2 beeinflußt maßgeblich den bei der Extraktion erzielbaren Wirkungsgrad der Entfernung der Produkte aus dem Brüdenkondensat.

Das in der Kolonne D2 gewonnene, wäßrige Rohprodukt wird in der Destillationskolonne 3 von den Verunreinigungen, wie $SO_2$, Methanol und Resten vom Undecan befreit.

Dabei wird bei einer Sumpftemperatur von 98°C ein Azeotrop aus diesen Stoffen mit geringen Wassermengen ausgetrieben, das nach der Kondensation im Scheider S3 in eine wäßrige, eine organische Phase und in nichtkondensierbare Dämpfe ($SO_2$, Methanol) zerfällt.

Die organische Phase wird dem Extraktionsmittel über einen Mischer (nicht dargestellt) wieder zugeführt, die wäßrige dem Extraktor R und die Dämpfe einem Verbrennungskessel.

Die Auftrennung des Gemisches aus Essigsäure, Ameisensäure, Furfurol und Wasser (mit den vorliegenden Konzentrationsverhältnissen) in seine reinen Komponenten ist durch gewöhnliche Destillationsvorgänge nur bedingt möglich. Es entsteht dabei immer ein Azeotrop, das aus Ameisensäure, Essigsäure und Wasser besteht.

Um alle Komponenten rein gewinnen zu können, muß man sich einer Schleppmitteldestillation bedienen. In der Literatur sind zahlreiche Schleppmittel angegeben, wie Äthylazetat, Diisopropyläther u. a. Als bestes Schleppmittel hat sich für das vorliegende Gemisch Di-n-propyläther und/oder Äthyl-n-butyläther erwiesen, welche eine Schleppwirkung von 10% Wasser aufweisen, während die bekanntesten Schleppmittel 4—5% Wasser im Azeotrop enthalten.

Das Schleppmittel wird dem Einsatzprodukt über eine Verhältnisregelung V zugegeben und in D4 als wasserhältiges Azeotrop über Kopf abdestilliert. Im Scheider S4 zerfällt das Azeotrop in seine Bestandteile, wobei nur geringfügige Mengen ineinander gelöst bleiben.

Das Sumpfprodukt aus D4 enthält somit nur mehr Spuren von Wasser und kann dann leicht in Kolonnen in die einzelnen Komponenten auf bekannte Art und Weise aufgetrennt werden. Das abdestillierte Wasser enthält geringe Verunreinigungen von Essigsäure, Ameisensäure, Furfurol und Schleppmittel. Das Schleppmittel wird dann vom Wasser abgestrippt. Das Wasser wird dem Extraktor R zugeführt.

## Patentansprüche

1. Verfahren zur Extraktion von Essigsäuren, Ameisensäure, gegebenenfalls Furfurol, u. dgl. aus einer wäßrigen Lösung von pflanzlichen Hydrolyseprodukten, wobei mit flüssigem, im wesentlichen wasserunlöslichem Trialkylphosphinoxid, das gegebenenfalls in einem organischen, im wesentlichen wasserunlöslichen Lösungsmittel gelöst ist, Essigsäure, Ameisensäure und gegebenenfalls das Furfurol u. dgl. bei einer Temperatur zwischen 40 und 100° C aus der wäßrigen Lösung extrahiert und von dem Extraktionsmittel durch Destillation getrennt werden, dadurch gekennzeichnet, daß ein Lösungsmittel für das Extraktionsmittel, das im Bereich von 170 bis 220° C bei Normaldruck siedet, verwendet wird, daß in einer ersten Stufe der überwiegende Teil des im beladenen Extraktionsmittel gelösten Wassers abdestilliert wird, und daß, vorzugsweise bei Unterdruck, in einer zweiten Stufe das Lösungsmittel für das Extraktionsmittel zusammen mit einer Mischung, vorzugsweise einem Azeotrop, mit Essigsäure, Ameisensäure, Wasser, gegebenenfalls Furfurol und Verunreinigungen abdestilliert wird und unbeladenes Extraktionsmittel abgezogen wird.

2. Verfahren nach Anspruch 1, dadurch gekennzeichnet, daß als Extraktionsmittel ein Trioctylphosphinoxid und als Lösungsmittel eine aliphatischer Kohlenwasserstoff, vorzugsweise n-Undecan, verwendet wird.

3. Verfahren nach Anspruch 1 oder 2, dadurch gekennzeichnet, daß eine 20 bis 80 gew.-%ige Lösung, vorzugsweise 30 bis 40 gew.-%ige, Lösung des Extraktionsmittels im Lösungsmittel verwendet wird.

4. Verfahren nach einem der Ansprüche 1 bis 3, dadurch gekennzeichnet, daß Methanol, gegebenenfalls mit weiteren Verunreinigungen von der von dem Extraktionsmittel abgetrennten Mischung mit Essigsäure, Ameisensäure, Wasser und gegebenenfalls Furfurol abdestilliert wird.

5. Verfahren nach einem der Ansprüche 1 bis 4, dadurch gekennzeichnet, daß dem Gemisch mit Essigsäure, Ameisensäure, Furfurol und Wasser ein Schleppmittel aus Di-n-propyläther und/oder Äthyl-n-butyläther für das Wasser zugesetzt wird, worauf das Wasser mit dem Schleppmittel abdestilliert wird.

## Claims

1. A process for the extraction of acetic acid, formic acid, where appropriate furfural, and the like from an aqueous solution of vegetable hydrolysis products, in which — by means of liquid, substantially water-insoluble trialkyl phosphine oxids which where appropriate is dissolved in an organic, substantially water-insoluble solvent — acetic acid, formic acid and where appropriate the furfural and the like are extracted from the aqueous solution at a temperature of between 40 and 100° C and are separated from the extraction medium by distillation, characterized in that a solvent is used for the extraction medium which boils in the range of 170 to 220° C at normal pressure, in a first stage the predominant part of the water dissolved in the charged extraction medium is distilled off in a first stage, and, preferably at underpressure, in a second stage the solvent for the extraction medium together with a mixture, preferably an azeotrope, with acetic acid, formic acid, water, where appropriate furfural and impurities is distilled off and uncharged extraction medium is removed.

2. A process according to claim 1, characterized in that a trioctyl phospine oxide is used as the extraction medium and a aliphatic hydrocarbon, preferably n-undecane, is used as the solvent.

3. A process according to claim 1 or 2, characterized in that a 20 to 80% by weight solution, preferably a 30 to 40% by weight solution, of the extraction medium in the solvent is used.

4. A process according to any of claims 1 to 3, characterized in that methanol, where appropriate with further impurities, is distilled off from the mixture separated from the extraction medium with acetic acid, formic acid, water, and where appropriate furfural.

5. A process according to any of claims 1 to 4, characterized in that a entrainer of di-n-propyl ether and/or ethyl-n-butyl ether for the water is added to the mixture with acetic acid, formic acid, furfural, and water, after which the water is distilled off with the entrainer.

**Revendications**

1. Procédé d'extraction d'acide acétique, acide formique, éventuellement furfural et produits analogues d'une solution aqueuse de produits d'hydrolyse végétaux, dans lequel, au moyen d'oxyde de trialkylphosphine liquide, essentiellement insoluble dans l'eau, qui est éventuellement dissous dans un solvant organique essentiellement insoluble dans l'eau, l'acide acétique, l'acide formique et éventuellement le furfural et produits analogues sont extraits de la solution aqueuse à une température comprise entre 40° et 100°C, et sont séparés par distillation du milieu d'extraction, caractérisé en ce qu'on utilise un solvant pour l'agent d'extraction que entre en ébullition dans la gamme de température de 170 à 220°C sous pression normale, que, dans un premier temps, la plus grande partie de l'eau dissoute dans l'agent d'extraction chargé est chassée par distillation et que dans un deuxième temps, de préférence sous vide partiel, le solvant pour l'agent d'extraction est chassé par distillation en même temps qu'un mélange, de préférence un azéotrope, comprenant de l'acide acétique, de l'acide formique, de l'eau, éventuellement du furfural et des impuretés, puis l'agent d'extraction non chargé est éliminé.

2. Procédé selon la revendication 1, caractérisé en ce qu'on utilise comme agent d'extraction un oxyde de trioctylphosphine et comme solvant un hydrocarbure aliphatique, de préférence n-undécane.

3. Procédé selon la revendication 1 ou 2, caractérisé en ce qu'on utilise une solution à 20 à 80% en poids, de préférence à 30 à 40% en poids de l'agent d'extraction dans le solvant.

4. Procédé selon une quelconque des revendications 1 à 3, caractérisé en ce que du méthanol éventuellement accompagné d'autres impuretés est chassé par distillation du mélange séparé de l'agent d'extraction contenant de l'acide acétique, de l'acide formique, de l'eau et éventuellement du furfural.

5. Procédé selon une quelconque des revendications 1 à 4, caractérisé en ce qu'on ajoute au mélange contenant acide acétique, acide formique, furfural et eau un produit d'entraînement pour l'eau constitué par de l'éther di-n-propylique et/ou de l'éther éthyle-n-butylique, puis l'eau est séparée par distillation avec le produit d'entraînement.